# DEMANDE DE BREVET EUROPEEN

(11) **EP 1 695 707 A1**
(43) Date de publication de la demande: **30.08.2006**
(21) Numéro de dépôt: 06114290.7
(22) Date de dépôt: 03.10.2002
(51) Int. Cl.: A61K 31/4436, A61K 47/10, A61K 47/14, A61K 47/30, A61P 17/06

(54) **Vernis à ongles contenant du tazarotène et son utilisation dans le traitement et/ou la prévention du psoriasis**

(30) Priorité: 05.10.2001 FR 0112825
(62) Demande divisionnaire de: 02800625.2
(71) Demandeur: Pierre Fabre Dermo-Cosmétique, 92100 Boulogne-Billancourt (FR)
(72) Inventeur: Lefrancois, Pascal, 81500 Lavaur (FR); Delaunois, Marlène, 31290 Villefranche du Laugarais (FR); Navarro, Roger, 09100 Pamiers (FR)
(74) Mandataire: Ahner, Francis

(57) **Abrégé**

La présente invention a pour objet l'utilisation du tazarotène pour la préparation d'un vernis à ongles pour le traitement et/ou la prévention du psoriasis, un vernis à ongles contenant du tazarotène et un procédé de fabrication.

L'invention concerne plus particulièrement un vernis à ongles contenant outre le tazarotène un agent filmogène soluble ou insoluble dans les solvants polaires et un ou des solvants physiologiquement acceptables. Il contient optionnellement un agent de pénétration.

## Description

La présente invention a pour objet l'utilisation du tazarotène pour la préparation d'un vernis à ongles pour le traitement et/ou la prévention du psoriasis, un vernis à ongles contenant du tazarotène et un procédé de fabrication.

Le psoriasis est une maladie de la peau qui touche plus de deux pour cent des populations occidentales.

Elle se traduit par une dermatose érythématosquameuse chronique. La lésion associe des anomalies du kératinocyte (activité mitotique fortement augmentée et différenciation anormale) à des phénomènes inflammatoires du derme et de l'épiderme.
La maladie est sous la dépendance de facteurs génétiques révélés par divers facteurs de l'environnement.

Les affections des ongles dues au psoriasis sont des formes de maladies tenaces qu'il n'a pas été possible jusqu'à présent de traiter de façon satisfaisante. Elles touchent, selon les pays entre 10 et 78% des populations développant un psoriasis.

Aujourd'hui, le traitement le plus répandu du psoriasis de l'ongle consiste en des injections sous-cutanées de corticoïdes. Ces corticoïdes sont des principes actifs anti-inflammatoires largement utilisés pour les traitements dermatologiques.

Ces injections très douloureuses peuvent être accompagnées d'un traitement topique.

Un autre traitement consiste à appliquer sur les ongles localement des substances spécifiques à action antipsoriasique sous forme de crème. Dans ce domaine, on a essayé les méthodes de traitement les plus diverses. Ainsi, dans un traitement combiné on a d'abord traité les ongles avec des solutions de substances à action antipsoriasique et on a appliqué des pansements avec de la crème pendant la nuit. Cette méthode de traitement est également fort désagréable et psychologiquement éprouvante pour les patients. D'une part, le traitement des ongles avec des solutions est nécessaire plusieurs fois par jour. D'autre part, ceux-ci doivent être munis de pansements, surtout la nuit. En conséquence, le traitement, qui dure habituellement plusieurs mois, n'est pas fréquemment poursuivi par les patients qui au contraire se découragent et deviennent négligents. Cela conduit à l'échec du traitement. Dans cette méthode, le succès du traitement est en outre compromis par le fait que les solutions et les crèmes sont habituellement miscibles à l'eau ou hydrophiles, et peuvent être éliminées de la surface de l'ongle ou entraînées par dissolution hors de l'ongle lors de la toilette, de bains et de douches, et doivent donc être ensuite appliquées à nouveau.

Le tazarotène est un dérivé rétinoïde connu pour son activité antipsoriasique.

Il s'agit d'un dérivé de rétinoïde également nommé 6-[(3,4-Dihydro-4,4-diméthyl-2H-1-benthiopyran-6-yl)éthynyl]-3-pyridinecarboxylate d'éthyle de formule

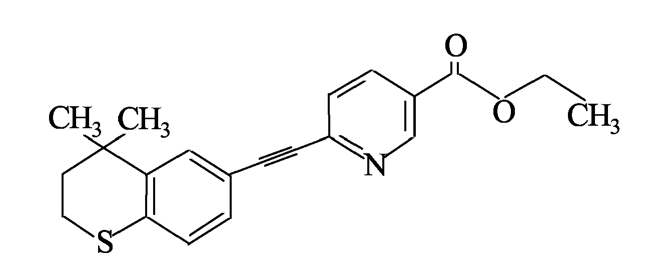

Il peut notamment se présenter sous forme de crème ou de gel.
Cependant si l'efficacité du tazarotène sur des plaques de psoriasis a été bien démontrée, traiter un psoriasis qui se développe sous l'ongle est délicat.
Il est difficile d'atteindre des concentrations efficaces de principe actif pour éliminer le psoriasis dans ces conditions.

On a maintenant trouvé qu'il était possible de traiter des psoriasis des ongles avec succès et/ou de les prévenir en appliquant sur les ongles, et notamment sur les ongles atteints de psoriasis, le vernis à ongles selon l'invention.

La présente invention a donc pour objet l'utilisation de tazarotène pour la préparation d'un vernis à ongles pour le traitement et/ou la prévention du psoriasis.
Un deuxième objet de l'invention est un vernis à ongles à action antipsoriasique contenant du tazarotène.
Le vernis à ongles selon l'invention contient avantageusement un agent filmogène soluble ou insoluble dans les solvants polaires.
La présente invention a aussi pour objet un vernis à ongles contenant outre du tazarotène et un agent filmogène soluble ou insoluble dans les solvants polaires,
a) un ou des solvants physiologiquement acceptables adaptés au type d'agent filmogène utilisé,
b) optionnellement un ou des agents de pénétration ainsi qu'éventuellement,
c) des additifs utilisés habituellement dans des cosmétiques.

Selon l'invention le vernis à ongles doit contenir suffisamment de principe actif afin que son dépôt sur l'ongle permette une libération convenable assurant une diffusion jusqu'au lit de l'ongle et permettant ainsi d'atteindre le seuil thérapeutique.

Un autre objet de la présente invention est un procédé de fabrication du vernis à ongles selon l'invention qui comprend une étape de mélange d'un agent filmogène et de tazarotène. Avantageusement, lorsque le vernis à ongles, objet de la présente invention contient d'autres constituants a), b) et/ou c) cités ci-dessus, que le principe actif et l'agent filmogène, on effectue la dissolution du principe actif et des autres constituants dans le ou les solvants avant l'agent filmogène.
Les vernis à ongles selon l'invention peuvent contenir des agents filmogènes solubles dans des solvants hydroalcooliques ou des solvants non polaires qui après séchage sur l'ongle, forment des films résistants à l'eau.

Conviennent en tant qu'agents filmogènes solubles dans les solvants non polaires (ou insoluble dans les solvants polaires), des substances à base de nitrate de cellulose ou de polymères synthétiques bien connus du formulateur pour leur innocuité. On peut citer par exemple, le polyacrylate de vinyle et le polyacrylate de vinyle partiellement saponifié, des copolymères d'acétate de vinyle d'une part et d'autre part, d'acide acrylique ou d'acide crotonique ou d'un maléate de monoalkyle, des copolymères ternaire d'acétate de vinyle d'une part et d'autre part, d'acide crotonique et de néodécanoate de vinyle ou d'acide crotonique et de propionate de vinyle, des copolymères d'éther méthyl vinylique et d'un maléate de monoalkyle, en particulier sous forme de maléate de monobutyle, des copolymères d'esters vinyliques d'acides gras et d'acide acrylique ou d'acide méthacryliques, des copolymères de N-vinylpyrrolidone, acide méthacrylique et méthacrylate d'alkyle, des copolymères d'acide acrylique et d'acide méthacrylique, ou d'acrylate d'alkyle ou de méthacrylate d'alkyle, des polyacétals de vinyle et des poly butyrals de vinyle, des poly-N-vinylpirrolydones substitués par des groupes alkyles des esters alkyliques de copolymères d'oléfine et d'anhydride maleïque et des produits de réaction de colophane et d'acide acrylique. Dans les esters, les restes d'alkyle sont habituellement des chaînes courtes et ne contiennent en général pas plus de quatre atomes de carbone.

Conviennent en tant qu'agents filmogènes solubles dans des solvants polaires les polyacrylamides; les copolymère acrylique/méthacrylique, polymethacrylate/butylacrylate, acrylique/acrylate; l'alcool polyvinylique; le copolymère polyvinylméthyl ether/anhy-dride maléïque; la polyvinylpyrrolidone; le copolymère polyvinylpyrrolidone/vinyl acétate et le copolymère vinylpyrrolidone/diméthylaminethyl méthacrylate.

Un mélange d'agents filmogènes de la même classe (soluble dans les solvants polaires ou bien soluble dans les solvants non polaires) peut être utilisé à titre d'agent filmogène dans le cadre de l'invention.

On préfère parmi les agents filmogènes solubles dans les solvants polaires les copolymères acryliques/acrylates et acrylique/méthacrylates ou le ter copolymère acrylique/acrylate/méthacrylate.

On préfère parmi les agents filmogènes solubles dans les solvants non polaires, le copolymère éther méthyl vinylique/maléate de monobutyle.

Parmi les solvants physiologiquement acceptables et à titre de solvants non polaires on peut citer des substances telles que des hydrocarbures, des hydrocarbures halogénés, alcools, éthers, cétones et esters, utilisés couramment dans des cosmétiques en particulier des esters d'acide acétique et de mono alcools, tels que l'acétate d'éthyle et l'acétate de butyle éventuellement en mélange avec des hydrocarbures aromatiques, tels que le toluène, et/ou des alcools tels que l'éthanol ou l'alcool isopropylique.

Lorsqu'on utilise à titre d'agent filmogène un agent filmogène soluble dans l'eau, on peut utiliser comme solvant, dans le cadre de la présente invention, des alcools tels que l'éthanol, l'alcool isopropylique ou tout autre alcool ayant un point d'ébullition suffisamment bas pour avoir un temps de séchage très court. Ces alcools peuvent être présents dans le vernis sous forme de mélanges et une quantité d'eau allant de 0 à 20% en poids par rapport à la quantité totale de solvant peut être alors ajoutée. Il s'agit des solvants dits solvants hydroalcooliques.

Dans le cadre de la présente invention et que l'agent filmogène soit soluble ou insoluble dans l'eau, on préfère utiliser une association de solvants ou système de solvants. Ces solvants ont une importance prépondérante pour le temps de séchage, la facilité d'application au pinceau et d'autres propriétés importantes du vernis ou de la pellicule de vernis. Le système de solvants peut être compris entre 70 et 94% en poids par rapport au poids total du vernis.

Ce système de solvant est de préférence un mélange de solvants à bas point d'ébullition (= point d'ébullition jusqu'à 100 °C) et de solvants à point d'ébullition moyen (= point d'ébullition jusqu'à 150°C), éventuellement avec une faible proportion de solvants à point d'ébullition élevé (= point d ébullition jusqu'à 200°C) .

A titre d'agents de pénétration on peut notamment citer l'urée, l'acide oléïque et l'éthoxy diglycol. Tout autre agent de pénétration utilisé couramment dans ce type de formulations pourra l'être également dans le cadre de la présente invention.

Les vernis à ongles selon l'invention peuvent contenir en outre des additifs utilisés couramment dans des cosmétiques, tels que plastifiants à base de phtalate, d'urée ou de camphre, de colorants ou de pigments colorés, agent nacrant, retardateurs de sédimentation, résines sulfonamide, silicates, parfums, agents mouillants, tels que le dioctylsulfosuccinate de sodium, dérivés de la lanoline, agents de protection contre la lumière, tels que la 2-hydroxy-4-méthoxybenzophénone, substances à action antibactérienne et substances à action kératolytique et/ou kératoplastique, telles que le sulfite d'ammonium, des esters et sels de l'acide thioqlycolique, l'urée, l'allantoïne, des enzymes et l'acide salicylique.
Les vernis à ongles colorés ou pigmentés offrent par exemple l'avantage que la composition selon l'invention peut être adaptée au sens esthétique du patient.

La préparation du vernis à ongles est effectuée de la façon usuelle par mélange des composants individuels et, si nécessaire un traitement ultérieur adapté à chaque composition.

Dans le vernis à ongles selon l'invention, le tazarotène est en général contenu en une quantité allant de 0,1 à 4%, de préférence de 0,1% à 2% en poids par rapport au poids total de la composition.

Le vernis à ongles selon la présente invention contient en général de 0,1 à 30%, de préférence de 5 à 20% en poids d'agent filmogène, de 69 à 99% en poids, de préférence de 79 à 94% et plus précisément de 81 à 86% en poids de solvant et de 0,5 à 15% en poids, de préférence de 1 à 10% et plus précisément de 3 à 7% d'agent de pénétration par rapport au poids total du vernis.

Les proportions citées ci-dessus permettent notamment d'obtenir un vernis adapté pour la chaîne de production tout en présentant des propriétés de facilité à l'étalement et un séchage rapide.

Il est connu que les couches cornées superficielles ont, entre autres, la fonction biologique d'empêcher la pénétration de substances étrangères. Les compositions selon l'invention permettent le passage en proportion considérable du principe actif à travers les 168 couches cornées superficielles et exercent ainsi une action durable en profondeur.

La présente invention concerne par ailleurs, à titre de mode particulier de réalisation, un vernis tel que décrit précédemment contenant en outre un corticoïde ou un mélange de différents corticoïdes.

Les corticoïdes pouvant être associés au tazarotène peuvent être choisis parmi les corticoïdes de :
- très forte activité, parmi lesquels le propionate de clobétasol ou le dipropionate de bétaméthasone ;
- forte activité, parmi lesquels : le valérate ou dipropionate de bétaméthasone, l'acétonide de fluocinolone et l'acéponate ou butyrate d'hydrocortisone ;
- assez forte activité, parmi lesquels : l'acétonide de fluocinolone, le désonide ;
- activité modérée, parmi lesquels: l'acétate d'hydrocortisone.

On préfère, à titre de vernis à ongles contenant un corticoïde, un vernis à ongles contenant le clobétasol ou un de ses sels pharmaceutiquement acceptable, tel que le propionate de clobétasol.

Cette classification des corticoïdes est européenne et non limitative pour l'invention. La quantité de corticoïde peut varier de 0,01% à 5% en poids par rapport au poids total du vernis en fonction de la gravité du psoriasis à traiter.

La présente invention est illustrée plus en détail à l'aide de l'exemple qui suit.

Exemple : Etude de l'activité de 6 formulations de vernis selon l'invention.

Dans les essais sur le pouvoir de pénétration, on a testé plusieurs formules préparées soit avec des agents filmogènes à tendance hydrophile dissous dans des milieux hydroalcooliques, soit avec des agents filmogènes à tendance hydrophobe dissous dans des solvants très peu polaires.
Dans les deux cas nous nous sommes préoccupés de la non solubilisation après séchage dans l'eau du film formé ce qui entraînerait au cours de la toilette la disparition du principe actif et donc la perte de l'activité du produit.

**Une première série d'essais** a été menée sur des explants de peau afin de vérifier sur ce modèle classique, si nous arrivions à discriminer plusieurs formules différentes.

Sur de la peau abdominale humaine dermatomée à 400 microns, en utilisant des cellules de diffusion de type cellules de Franz modifiées, nous avons appliqué les différentes formules en doses finies soit 9,2 mg/cm². L'étude de passage s'est effectuée sur 24h. Le principe actif tazarotène a diffusé à travers la peau et a été collecté dans le liquide récepteur à 3h, 6h, 12h et 24h.

Au bout de 24h, l'actif a été dosé dans le tissu, à la fin de la phase de diffusion (derme et épiderme) et à la surface du tissu, après avoir nettoyé en surface le vernis.

Deux types de formules ont été testés :

| | Formulaires polaires (en %) | | | Formulaires apolaires (en %) | | | |
|---|---|---|---|---|---|---|---|
| | 1 | 2 | 3 | 4 | 5 | 6 | Temoin gel |
| TAZAROTENE | 0,5 | 0,5 | 0,1 | 1 | 0,5 | 0,1 | 0,1 |
| B.H.T | 0,05 | 0,05 | 0,05 | 0,05 | 0,05 | 0,05 | - |
| UREE | - | 5 | - | - | - | - | - |
| COPOLYMERE: | 14 | 14 | 14 | - | - | - | - |
| ACIDE ACRYLIQUE/ METHYL METHACRYLATE/ ETHYL ACRYLATE | | | | | | | |
| EAU PURIFIEE | 9,99 | 9,41 | 10,04 | - | - | - | - |
| ETHANOL | 75,46 | 71,04 | 75,81 | - | - | - | - |
| COPOLYMERE: ETHER METHYL VINYLIQUE MONOBUTYL ESTER D'ACIDE MALEIQUE | - | - | - | 15 | 15 | 15 | - |
| ISOPRANOL | - | - | - | 50,15 | 50,45 | 50,69 | - |
| ACETATE D'ETHYLE | - | - | - | 33,80 | 34,00 | 34,16 | - |

B.H.T : Butyl hydroxy toluene
Avec solvant et agent filmogène polaire (formules 1, 2 et 3)
Avec solvant et agent filmogène non polaire (formules 3, 4 et 5)
Nous avons utilisé un témoin du commerce, connu sous la marque ZORAC® , gel dermique dosé à 0,1% de tazarotène.

Etude du passage transcutané sur 24 heures du tarazotène.

| | Caractéristique | Passage en micro grammes |
|---|---|---|
| Formule 1 | Vernis hydrophile tarazotène 0,5% Urée 5% | 0,175 |
| Formule 2 | Vernis lipophile tarazotène 1% | 0,12 |
| Formule 3 | Vernis lipophile tazazotène 0,5% | 0,065 |
| Formule 4 | Vernis hydrophile tarazotène 0,5% | 0,05 |
| Formule 5 | Gel tarazotène 0,1% | 0,012 |

Résultats : Nous avons démontré :
- que plus la concentration en actif est importante, plus le principe actif pénètre,
- quel que soit le solvant et le type d'agent filmogène, le principe actif au même dosage pénètre de la même façon,
- que l'ajout d'urée favorise le passage trans ongle dans le cas de la formule de type polaire.

**Une deuxième série d'essais** de pénétration a été conduite sur la corne de boeuf.
Ces cornes de boeufs ont été découpées de façon à obtenir des disques de kératine de diamètre compatible avec celui des cellules de Franz, ces disques ayant une épaisseur standardisée à 0,6 mm.

La cellule de Franz utilisée dans cette expérimentation se présente comme un récipient à double compartiment séparé par la peau ou la corne. Dans le compartiment inférieur, se trouve une solution de sérum physiologique thermostatée à 37°C. Le produit à étudier est déposé dans le récipient supérieur, au contact de la peau ou de la corne. Des prélèvements réguliers sont effectués dans le compartiment inférieur, pour étudier les quantités de principe actif.

Le vernis contenant le tazarotène est déposé sur ce disque de corne et le passage trans ongle est suivi en utilisant du tazarotène marqué au C14. La méthode d'étude du pouvoir de pénétration sur de la corne de boeuf permet d'étudier les composés en ce qui concerne leur pouvoir de pénétration à une concentration efficace vis-à-vis de la corne dont la composition est très semblable à la composition de l'ongle.

Sur la corne de boeuf sont déposés 7,1 mg/cm² de vernis tels que définis dans les formules de 1 à 6.

Le temps de contact a été de 48 heures avec des prélèvements à 6h, 12h, 24h, 36h et 48h. L'ongle à été dermatomé en lames de 40 microns, la surface de la corne et la base de la corne, faisant 240 microns.

Etude du passage trans ongle de 24 heures du tarazotène.

| | Caractéristique | Passage en micro grammes |
|---|---|---|
| Formule 1 | Vernis hydrophile tarazotène 0,5% | 0,001 |
| Formule 2 | Vernis hydrophile tarazotène 0,5% | 0,05 |
| | Urée | |
| Formule 3 | Vernis lipophile tazazotène 1% | 2 |
| Formule 4 | Vernis lipophile tarazotène 0,5% | 0,025 |

Les résultats ont été les mêmes. Le produit avec solvant polaire et qui contient l'urée a un meilleur passage trans ongle.

**En conclusion :** Les vernis à ongles contenant un agent filmogène et un solvant selon l'invention, qu'ils soient à base de solvants polaires et d'agents filmogènes du même type avec ou sans urée, ou qu'ils soient à base de solvants non polaires, ont un passage à travers l'ongle qui permet d'atteindre le seuil thérapeutique.

Ces vernis permettent d'avoir une efficacité rapide et indolore contrairement aux autres thérapies connues dans cette affection.

## Revendications

1. Utilisation du tazarotène pour la préparation d'un vernis à ongles pour le traitement du psoriasis.

2. Vernis à ongles à action antipsoriasique contenant du tazarotène.

3. Vernis à ongles à action antipsoriasique selon la revendication 2, **caractérisé en ce qu'**il contient en outre un agent filmogène soluble ou insoluble dans les solvants polaires.

4. Vernis à ongles à action antipsoriasique selon la revendication 3, **caractérisé en ce qu'**il contient outre du tazarotène et un agent filmogène, un ou des solvants physiologiquement acceptables adaptés au type d'agent filmogène utilisé.

5. Vernis à ongles à action antipsoriatique selon la revendication 4, **caractérisé en ce qu'**il contient un ou des agents de pénétration, ainsi qu'éventuellement des additifs utilisés habituellement dans des cosmétiques.

6. Vernis à ongles selon la revendication 5, **caractérisé en ce que** l'agent de pénétration est choisi parmi l'urée, l'acide oléïque et l'étoxy diglycol.

7. Vernis à ongles selon l'une quelconque des revendications 2 à 6, **caractérisé en ce qu'**il contient le tazarotène en une quantité allant de 0,1 à 4% en poids par rapport au poids total de la composition.

8. Vernis à ongles selon la revendication 7, **caractérisé en ce qu'**il contient le tazarotène en une quantité allant de 0,1% à 4% en poids par rapport au poids total de la composition.

9. Vernis à ongles selon l'une quelconque des revendications 3 à 8, **caractérisé en ce que** l'agent filmogène soluble dans les solvants non polaires est choisi parmi le nitrate de cellulose, le polyacrylate de vinyle et le polyacrylate de vinyle partiellement saponifié, des copolymères d'acétate de vinyle d'une part et d'autres part, d'acide acrylique ou d'acide crotonique ou d'un maléate de monoalkyle, des copolymères ternaire d'acétate de vinyle d'une part et d'autre part, d'acide crotonique et de néodécanoate de vinyle ou d 'acide crotonique et de propionate de vinyle, des copolymères d'éther méthyl vinylique et d'un maléate de monoalkyle, en particulier sous forme de maléate de monobutyle, des copolymères d'esters vinyliques d'acides gras et d'acide acrylique ou d'acide méthacryliques, des copolymères de N-vinylpyrrolidone, acide méthacrylique et méthacrylate d'alkyle, des copolymères d'acide acrylique et d'acide méthacrylique, ou d'acrylate d'alkyle ou de méthacrylate d'alkyle, des polyacétals de vinyle et des poly butyrals de vinyle, des poly-N-vinylpirrolydones substitués par des groupes alkyles des esters alkyliques de copolymères d'oléfine et d'anhydride maleïque et des produits de réaction de colophane et d'acide acrylique.

10. Vernis à ongles selon l'une quelconque des revendications 3 à 8, **caractérisé en ce que** l'agent filmogène soluble dans les solvants polaires est choisi parmi les polyacrylamides; les copolymères acrylique/méthacrylique, olymethacrylate/butylacrylate, acrylique/acrylate; l'alcool polyvinylique; le copolymère polyvinylméthyl éther/anhydride maléïque; la polyvinylpyrrolidone; le copolymère polyvinylpyrrolidone/vinyl acétate et le copolymère vinylpyrrolidone/diméthylaminethyl méthacrylate.

11. Procédé pour la préparation d'un vernis à ongles selon l'une quelconque des revendications 3 à 9, **caractérisé en ce que** qu'il contient une étape de mélange d'un agent filmogène soluble ou insoluble dans l'eau avec le tazarotène ainsi qu'éventuellement avec d'autres composants usuels pour la préparation de vernis à ongles.

12. Vernis à ongles selon l'une quelconque des revendications 3 à 10, **caractérisé en ce qu'**il contient en outre un corticoïde ou un mélange de différents corticoïdes.

13. Vernis à ongles selon la revendication 12, **caractérisé en ce que** le corticoïde peut être choisi parmi le propionate de clobétasol ou le dipropionate de bétaméthasone, le valérate ou dipropionate de bétaméthasone, l'acétonide de fluocinolone et l'acéponate ou butyrate d'hydrocortisone, l'acétonide de fluocinolone, le désonide, l'acétate d'hydrocortisone.

14. Vernis à ongles selon la revendication 13, **caractérisé en ce que** le corticoïde est le clobétasol ou un de ses sels pharmaceutiquement acceptable.
